# EUROPEAN PATENT APPLICATION

(11) **EP 1 900 757 A1**
(43) Date of publication of application: **19.03.2008**
(21) Application number: 06120744.5
(22) Date of filing: 15.09.2006
(51) Int. Cl.: C08F 4/64, C07C 215/18, C07F 7/00, C07C 31/38

(54) **Fluorinated dialkoxy-diimino catalyst components**

(71) Applicant: TOTAL PETROCHEMICALS RESEARCH FELUY, 7181 Seneffe (Feluy) (BE)
(72) Inventor: Carpentier, Jean-François, F-35690, Acigne (FR); Kirillov, Evgueni, 23 rue de Chatillon F-35000, Rennes (FR); Thomas, Christophe, F-22690, La Vicomte/ Rance (FR); Razavi, Abbas, B-7000, Mons (BE)

(57) **Abstract**

This invention relates to the fluorinated dialkoxy-diimine metal complexes and their use in catalyst system for the polymerisation or oligomerisation of ethylene and alpha-olefins.

## Description

The present invention discloses catalyst components based on fluorinated dialkoxy-diimino ligands, their method of preparation and their use in the polymerisation or oligomerisation of olefins.

Investigations to replace ubiquitous cyclopentadienyl-type ligands in modern coordination chemistry of early transition metals have become very popular in recent years. Hard, electronegative π-donor ligands such as aryloxides/alkoxides are attractive because they offer strong metal-oxygen bonds that are expected to stabilize complexes of these electropositive metals. Also, the great variety of these ligands conveniently obtained from alcohols allows considerable stereo-electronic variations. Accordingly, group 4 metal complexes of the type [MX₂(OZ)₂] and [MX₂(OZZO)], where OZ⁻ and OZZO²⁻ are monoanionic and chelating dianionic ligands respectively, have retained considerable attention. Most successful developments in terms of synthetic organometallic chemistry and catalysis have appeared using *aryloxide* (phenolate) ligands, with significant contributions of the groups of Fujita such as for example Matsui et al. (S. Matsui, M. Mitani, J. Saito, Y. Tohi, H. Makio, N. Matsukawa, Y. Takagi, K. Tsuru, M. Nitabaru, T. Nakano, H. Tanaka, N. Kashiwa, T. Fujita, in J. Am. Chem. Soc. 2001, 123, 6847-6856) or Kojoh et al. (S. Kojoh, T. Matsugi, J. Saito, M. Mitani, T. Fujita, N. Kashiwa, Chem. Lett. 2001, 822-823) or Saito et al. (J. Saito, M. Mitani, S. Matsui, J. Y. Mohri, S. Kojoh, N. Kashiwa, T. Fujita, Angew. Chem., Int. Ed. 2001, 40, 2918-2920; Angew. Chem. 2001, 113, 3002-3004) or Mitani et al. (M. Mitani, J. Mohri, Y. Yoshida, J. Saito, S. Ishii, K. Tsuru, S. Matsui, R. Furuyama, T. Nakano, H. Tanaka, S.-I. Kojoh, T. Matsugi, N. Kashiwa, T. Fujita, J. Am. Chem. Soc. 2002, 124, 3327-3336) or Saito et al. (J. Saito, M. Mitani, J. Mohri, S. Ishii, Y. Yoshida, T. Matsugi, S. Kojoh, N. Kashiwa, T. Fujita, T. Chem. Lett. 2001, 576-577) or Mitani et al. (M. Mitani, R. Furuyama, J.-I. Mohri, J. Saito, S. Ishii, H. Terao, N. Kashiwa, T. Fujita, J. Am. Chem. Soc. 2002, 124, 7888-7889) or of the group of Coates such as for example Tian and Coates (J. Tian, G. W. Coates, Angew. Chem., Int. Ed. 2000, 39, 3626-3629; Angew. Chem. 2000, 112, 3772-3775 or Tian et al. (J. Tian, P. D. Hustad, G. W. Coates, J. Am. Chem. Soc. 2001, 123, 5134-5135) or Mason and Coates (A. F. Mason, G. W. Coates, J. Am. Chem. Soc. 2004, 126, 16326-16327).

Comparatively, group 4 metal [MX₂(OZ)_{2]} and [MX₂(OZZO)] type complexes that incorporate simple (amino-)alkoxide ligands have been much less studied. This is mainly due to aggregation problems traditionally encountered with these relatively more basic ligands (as compared to aryloxides). A valuable approach to overcome this issue consists in introducing electron-withdrawing CF₃ groups α to the alkoxides; this generates increased intra- and inter-molecular repulsions and a much less basic alkoxide O-atom, and in turn a much reduced bridging ability. On this principle, Jordan et al. have developed "fluorous" group 4 complexes with two pyridine-alkoxide ligands such as for example in Tsukahara et al (T. Tsukahara, D. C. Swenson, R. F. Jordan, Organometallics 1997, 16, 3303-3313). Tetradentate diamino-dialkoxide {ONⁿNO}²⁻ ligands with a ethylene- (n = 2), propylene- (n = 3) or chiral 1,2-cyclohexyldiamine backbone flanked by two fluorinated tertiary alkoxides were recently prepared as well as neutral groups 3, 4 and 13 metal complexes derived thereof. They are described for example in Lavanant et al. (L. Lavanant, T.-Y. Chou, Y. Chi, C. W. Lehmann, L. Toupet, J.-F. Carpentier, Organometallics 2004, 23, 5450-5458) or in Amgoune et al. (A. Amgoune, L. Lavanant, C. M. Thomas, Y. Chi, R. Welter, S. Dagorne, J.-F. Carpentier, Organometallics, 2005, 24, 6279-6282) or in Kirillov et al. (E. Kirillov, L. Lavanant, C. M. Thomas, T. Roisnel, Y. Chi, J.-F. Carpentier, Chem. Eur. J. 2006, in press).

There is a demand for new catalyst systems having good activity and able to produce polymers or oligomers tailored to specific needs.

It is an aim of the present invention to prepare new catalyst components that can be used in the polymerisation or oligomerisation of olefins.

It is also an aim of the present invention to provide very active catalyst components.

It is another aim of the present invention to provide a method for homo- or co-polymerising or oligomerising olefins.

The present invention reaches, at least partially, any one of those aims.

Accordingly, the present invention discloses a class of fluorinated dialkoxy-diimine pro-ligands of formula 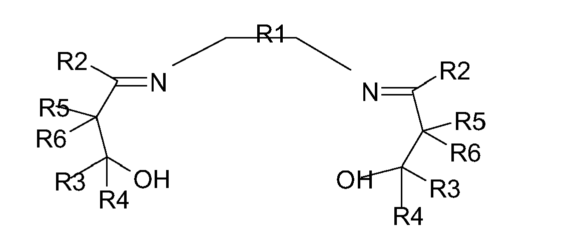 wherein
R¹ is a bridge having from 1 to 6 carbon atoms, substituted or unsubstituted and wherein the substituents if present may form a ring either with each other or with R²;
R² are each independently selected from hydrogen, alkyl, aryl or heteroaryl having from 1 to 20 carbon atoms;
R³ and R⁴ are each independently selected from CF₃, CₙF_{2n+1,} wherein n is an integer between 1 and 10, from aryl or alkyl having from 1 to 20 carbon atoms;
R⁵ and R⁶ are each independently selected from hydrogen, alkyl or aryl having from 1 to 20 carbon atoms.

Preferably both R² are the same and are alkyl or phenyl unsubstituted or substituted. The substituents, if present, can be selected from H, F, CF₃ or isopropyl. If the substituents are H, F or CF₃ they are located preferably at positions 2 and/or 3 and/or 4 and/or 5 and/or 6. If they are isopropyl, they preferably occupy positions 2 and 6.

Preferably R³ and R⁴ are the same and are CF₃ or CₙF₂ₙ₊₁, more preferably CF₃.

Preferably R⁵ and R⁶ are the same and are hydrogen.

The invention also discloses a metallic complex obtained by metallation of the fluorinated dialkoxy-diimine pro-ligand with a metal salt or precursor of formula MX₄
wherein M is a metal group 4 of the Periodic Table,
wherein each X is the same or different and is halogen, preferably chlorine, or alkyl or benzyl.

The solvent may be selected from dichloromethane (DCM) or tetrahydrofuran (THF) or a hydrocarbon such as toluene and the complexation reaction is carried out at a temperature of from 20 °C to reflux.

The amount of ligand is of one equivalent of ligand per metallic equivalent.

The present invention further discloses an active catalyst system comprising the metallic complex and an activating agent having an ionising action.

It can be aluminoxane and comprise oligomeric linear and/or cyclic alkyl aluminoxanes represented by formula 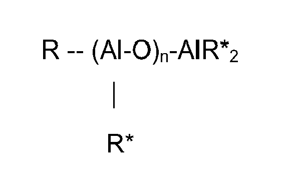 for oligomeric, linear aluminoxanes and by formula 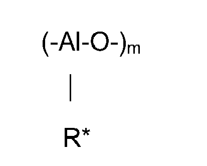 for oligomeric, cyclic aluminoxane,
wherein n is 1-40, preferably 10-20, m is 3-40, preferably 3-20 and R* is a C₁-C₈ alkyl group and preferably methyl.

The amount of activating is selected to give an Al/M ratio of from 100 to 3000, preferably of about 1000.

Suitable boron-containing activating agents may comprise a triphenylcarbenium boronate such as tetrakis-pentafluorophenyl-borato-triphenylcarbenium as described in EP-A-0427696, or those of the general formula [L'-H] + [B Ar₁ Ar₂ X₃ X₄]- as described in EP-A-0277004 (page 6, line 30 to page 7, line 7). The amount of boron -containing activating agent is selected to give B/M ratio of from 0.5 to 5, preferably of about 1.

In another embodiment, according to the present invention, the metallic complex may be deposited on a conventional support impregnated with an activating agent.
Preferably, the conventional support is silica impregnated with methylaluminoxane (MAO).

Alternatively, it can be an activating support such as fluorinated alumina silica.

The present invention further discloses a method for preparing an active catalyst system that comprises the steps of:
a) providing a fluorinated dialcohol-diimine pro-ligand;
b) complexing the pro-ligand of step a) with a metallic salt or precursor MX₄ in a solvent;
c) retrieving a catalyst component;
d) activating the catalyst component with an activating agent having an ionising action;
e) optionally adding a cocatalyst;
f) retrieving an active oligomerisation or polymerisation catalyst system.

Alternatively, in step d), the catalyst component is deposited on a support impregnated with an activating agent or on a fluorinated activating support.

The cocatalyst may be selected from triethylaluminium, triisobutylaluminum, tris-n-octylaluminium, tetraisobutyldialuminoxane or diethyl zinc.

The active catalyst system is used in the oligomerisation and in the homo- or copolymerisation of ethylene and alpha-olefins.

The present invention discloses a method for the oligomerisation or the homo- or copolymerisation of ethylene and alpha-olefins that comprises the steps of:
a) injecting the active catalyst system into the reactor;
b) injecting the monomer and optional comonomer;
c) maintaining under polymerisation conditions;
d) retrieving the oligomers and/or polymer.

The pressure in the reactor can vary from 0.5 to 50 bars, preferably from 5 to 25 bars.

The polymerisation temperature can range from 10 to 100°C, preferably from 50 to 85°C.

Preferably the monomer and optional comonomer are selected from ethylene, propylene, 1-hexene, 1-octene or styrene.

The present invention also discloses the polymers and oligomer compositions obtained with the new catalyst systems.

### List of figures.

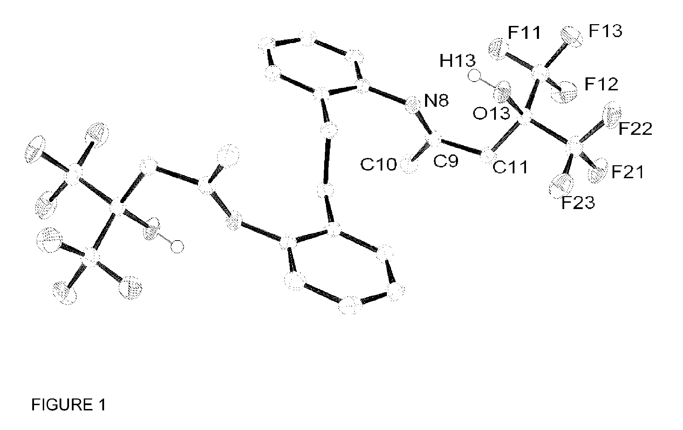

Figure 1 represents the crystal structure of pro-ligand 3.

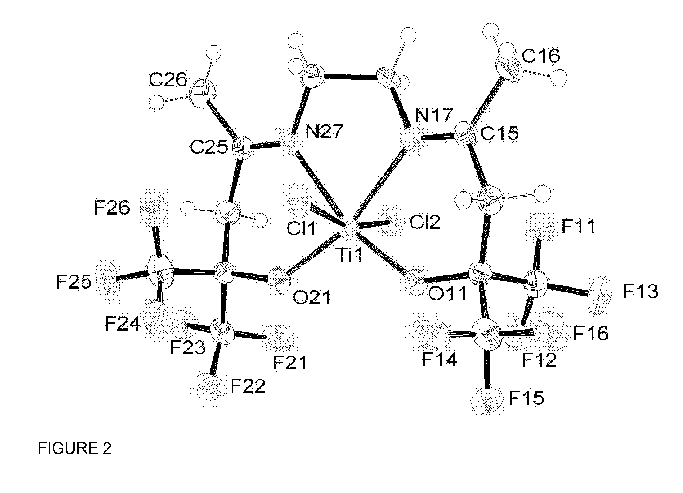

Figure 2 represents the crystal structure of complex 5.

### Examples.

### Synthesis of 1,1,1-trifluoro-4-[(2-{[4,4,4-trifluoro-3-hydroxy-1-methyl-3-(trifluoromethyl)butylidene]amino}ethyl)imino]-2-(trifluoromethyl)pentan-2-ol (1).

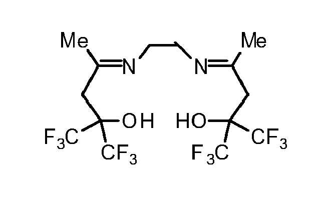

A Schlenk tube was charged with 0.80 g of montmorillonite, 10 mL of chloroform and 2.75 g (12.27 mmol) of 4,4,4-trifluoro-3-hydroxy-3-(trifluoromethyl)butan-2-one. Under an argon atmosphere, 0.36 g (6.13 mmol) of 1,2- ethylene-diamine (0.36 g, 6.13 mmol) were added and the reaction mixture was refluxed for a period of time of 72 h. The reaction mixture was filtered off and the filtrate was concentrated under reduced pressure. The resulting oily product was recrystallised from methanol at room temperature to give 3.88 g of **1** as an orange solid with a yield of 69%.
NMR results for **1** were as follows:
¹H NMR (500 MHz, CDCl₃, 298K): δ (ppm) 2.04 (s, 6H, Me), 2.73 (s, 4H, CH₂), 3.66 (s, 4H, CH₂N), 9.88 (s, 2H, OH). ¹³C NMR (75 MHz, CDCl₃, 298K): δ (ppm) 20.63 (CH₂), 33.41 (Me), 50.42 (CH₂-N), 121.1 (C-OH), 125.5 (C-F), 171.42 (C=N). ¹⁹F NMR (182 MHz, CDCl₃, 298K): δ (ppm) -78.9 (s, 12F).
Anal. Calcd. for C₁₄H₁₆F₁₂N₂O₂: C, 35.60; H, 3.41. Found: C, 36.44; H, 3.89.

### Synthesis of racemic 4,4'-(cyclohexane-1,2-diyibis(azan-1-yl-1-ylidene))bis(1,1,1-trifluoro-2-(trifluoromethyl)pentan-2-ol) (rac-trans-2).

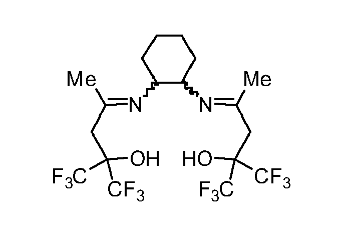

pro-Ligand **2** was prepared using the same procedure as that described for pro-ligand **1** starting from 0.80 g of montmorillonite, 2.50 g (11.1 mmol) of fluorinated aldol 4,4,4-trifluoro-3-hydroxy-3-(trifluoromethyl)butan-2-one and 0.63 g (5.5 mmol) of *trans*-1,2-diaminocyclohexane to give 3.97 g of **2** as a white solid with a yield of 68%. The melting temperature was of 108.4 °C.
NMR results for rac-**2** were as follows:
¹H NMR (500 MHz, CDCl_{3,} 298K): δ (ppm) 1.38-1.84 (m, 8H, cyclohexyl), 2.01 (s, 6H, Me), 2.65 (m, 2H, CH-N du cyclohexyl), 10.19 (s, 2H, OH).
¹³C NMR (75 MHz, CDCl_{3,} 298K): δ (ppm) 170,0 (2C, CN), 21.10 (2C, CH₂-C-CF₃), 24.20 (2C, CH₃), 32.02 (2C, CH₂ from cyclohexyl), 39.10 (2C, CH₂-CN from cyclohexyl), 64.30 (2C, CH-N de cyclohexyl), 122.0 (2C, CO), 126.0 (2C, q, J_{CF} = CF₃).
¹⁹F NMR (182 MHz, CDCl₃, 298K): δ (ppm) -78.72 (q, J = 10.5 Hz, 6F), -79.20 (q, J = 10.34 Hz, 6F).
HR-MS (70 eV, El): m/z calc. for C₁₈H₂₂F₁₂N₂O₂: 526.1490; found: 526.1521 (6 ppm). [*M*-*CF*₃]⁺ (C₁₇H₂₂N₂O₂F₉); theor. mol. weigh: 457.1538; found: 457.1568.

### Synthesis of 4,4'-((1 R,2R)-cyclohexane-1,2-divibis(azan-1-yl-1-ylidene))bis(1,1,1-trifluoro-2-(trifluoromethyl)pentan-2-ol) (R,R-2).

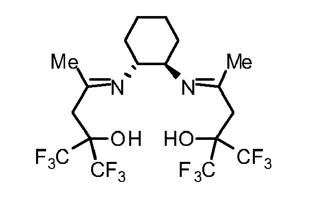

(R,R)-diol **2** was prepared using a procedure similar to that used to prepare racemic diol 2, starting from 1.20 g of montmorillonite, 4.24 g (18.95 mmol) of fluorinated aldol 4,4,4-trifluoro-3-hydroxy-3-(trifluoromethyl)butan-2-one and 0.63 g (5.5 mmol) of (1*R*,2*R*)-diaminocyclohexane to give 6.97 g of **(*R,R*)-2** with a yield of 70%.
NMR results for **(*R,R*)-2** were as follows:
¹H NMR (200 MHz, CDCl₃, 298K): δ (ppm) 1.42-1.85 (m, 8H, cyclohexyl), 2.02 (s, 6H, 2Me), 2.66 (s, 4H, cyclohexyl), 3.57 (m, 2H, CH-N), 10.19 (s, 2H, OH).
¹⁹F NMR (182 MHz, CDCl₃, 298K): δ (ppm) -78.74 (q, J = 10.35 Hz, 6F), -79.22 (q, J = 10.35 Hz, 6F).

### Synthesis of 4,4'-(2,2'-(ethane-1,2-divl)bis(2, 1-phenviene))bis(azan-1-yl-1-ylidene)bis(1,1,1-trifluoro-2-(trifluoromethyl)pentan-2-ol) (3).

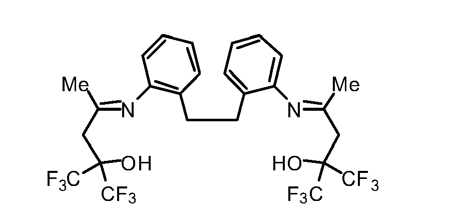

In a 50 mL flask, a mixture of 3.00 g (13.39 mmol) of fluorinated aldol 4,4,4-trifluoro-3-hydroxy-3-(trifluoromethyl)butan-2-one and 0.95 g (4.48 mmol) of 2-[2-(2-aminophenyl)ethyl]phenylamine in 40 mL of toluene, in the presence of a catalytic amount of paratoluenesulfonic acid (PTSA), was refluxed for 100 h using a Dean-Stark apparatus. The reaction mixture was filtered off, the filtrate was evaporated in vacuum and the residue was recrystallised from a CH₂Cl₂/toluene mixture to give 1.10 g of **3** as colourless crystals with a yield of 39%.
NMR results for **3** were as follows:
¹H NMR (200 MHz, CD₂Cl₂, 298K): δ (ppm) 1.95 (s, 6H, CH₃), 2.70 (s, 4H, CH₂), 2.97 (s, 4H, CH₂), 6.62 (m, 2H, arom.), 7.19 (m, 6H, arom.), 9.35 (s, 2H, OH). ¹⁹F NMR (188 MHz, CD₂Cl₂, 298K): δ (ppm) -78.9.
Anal. Calcd. for C₂₆H₂₄F₁₂N₂O₂: C, 50.01; H, 3.87. Found: C, 51.00; H, 4.28.

### Synthesis of [OC(CF₃)₂CH₂C(CH₃)=NCH₂CH₂N=C(CH₃)CH₂C(CF₃)₂O]Zr(CH₂Ph)₂ (4).

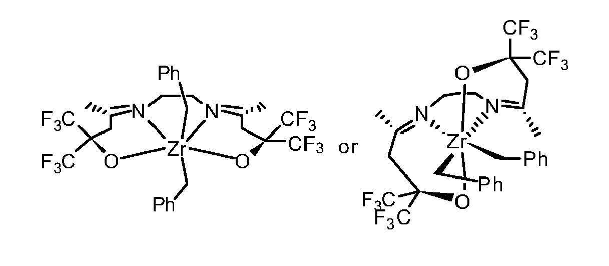

1) NMR-scale synthesis: A Teflon-valved NMR tube was charged with 25.7 mg (54.42 µmol) of diol **1** and 24.8 mg (54.42 µmol) of Zr(CH₂Ph)₄. About 0.5 mL of dry toluene-d₈ were added under vacuum. The tube was kept for a period of time of 3 to **4** hours at a temperature of -30 °C and NMR spectra were recorded. The formation of **4** proceeded with a yield of about 95%, as determined by ¹H NMR.
2) Preparative synthesis: to a solution of 0.42 g (0.86 mmol) of fluorinated aldol 4,4,4-trifluoro-3-hydroxy-3-(trifluoromethyl)butan-2-one in 4 mL of toluene, was added a solution of 0.41 g (0.89 mmol) of Zr(CH₂Ph)₄ in 3 mL of toluene at a temperature of -30 °C under vigorous stirring. The reaction mixture was kept at a temperature of -30 °C overnight, after which 4 precipitated as a yellow crystalline solid. The product was separated and dried under vacuum to give 0.25 g of **4** with a yield of 38 %.
   NMR results for **4** were as follows:
   ¹H NMR (500 MHz, CD₂Cl₂, 298K): δ (ppm) 2.13 (s, 4H, ZrCH₂Ph), 2.16 (s, 6H, CH₃), 2.89 (s, 4H, OC(CF₃)₂CH₂), 3.66 (s, 4H, NCH₂), 6.68 (d, ³J = 7.5 Hz, 4H, o-Ph), 6.75 (t, ³J = 7.5 Hz, 2H, p-Ph), 7.04 (m, ³J = 7.5 Hz, 4H, m-Ph).
   ¹³C{¹H} NMR (125 MHz, CD₂Cl₂, 298K): δ (ppm) 21.1 (CH₂Ph), 24.0 (CH₃), 40.6 ((CF₃)₂CH₂), 51.3 (NCH₂), 79.5 (C(CF₃)₂), 119.9 (p-Ph), 123.8 (C(CF₃)₂), 125.5 (o-Ph), 127.6 (m-Ph), 151.1 (i-Ph), 177.5 (N=C(CH₃)).
   ¹⁹F NMR (188 MHz, CD₂Cl₂, 298K): δ (ppm) -76.9. Anal. Calcd. for C₂₈H₂₈F₁₂N₂O₂Zr: C, 45.22; H, 3.79. Found: C, 45.77; H, 3.84.

### Synthesis of [OC(CF₃)₂CH₂C(CH₃)=NCH₂CH₂N=C(CH₃)CH₂C(CF₃)₂O]TiCl₂ (5).

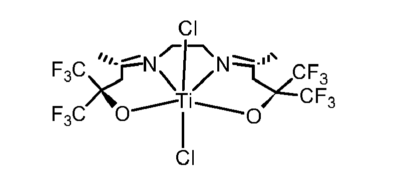

To a solution of 100 mg (0.21 mmol) of diol **1** in 4 mL of toluene, was added a solution of 20.1 mg (0.105 mmol) of TiCl₄ and 30.1 mg (0.105 mmol) of Ti(O*ⁱ*Pr)₄ in 4 mL of toluene at a temperature of -30 °C. The reaction mixture was kept at a temperature of -30 °C overnight, and colourless crystals precipitated. The crystals were separated, washed with a minimal amount of toluene and dried under vacuum to give 70 mg of **5** with a yield of 56 % .
NMR results for **5** were as follows:
¹H NMR (500 MHz, CD₂Cl₂, 298K): δ (ppm) 2.41 (s, 6H, CH₃), 3.66 (s, 4H, C(CF₃)₂C*H*₂), 4.20 (s, 4H, NCH₂). ¹³C{¹H} NMR (125 MHz, CD₂Cl₂, 298K): δ (ppm) 23.6 (CH₃), 41.8 ((CF₃)₂CH₂), 46.7 (NCH₂), 85.3 (C(CF₃)₂), 122.2 (C(CF₃)₂), 173.9 (N=C(CH₃)).
¹⁹F NMR (188 MHz, CD₂Cl₂, 298K): δ (ppm) -76.2.
Anal. Calcd. for C₁₄H₁₄Cl₂F₁₂N₂O₂Ti: C, 28.55; H, 2.40. Found: C, 28.96; H, 2.59.

## Claims

1. A fluorinated dialcohol-diimine pro-ligand of general formula 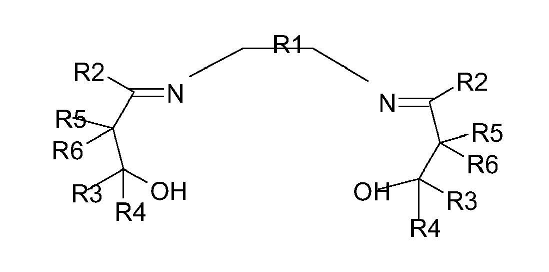 wherein
R¹ is a bridge having from 1 to 6 carbon atoms, substituted or unsubstituted and wherein the substituents if present may form a ring either with each other or with R²;
R² are each independently selected from hydrogen, aryl or alkyl having from 1 to 20 carbon atoms;
R³ and R⁴ are each independently selected from CF₃, CₙF_{2n+1,} wherein n is an integer between 1 and 10, from aryl or alkyl having from 1 to 20 carbon atoms;
R⁵ and R⁶ are each independently selected from hydrogen, alkyl or aryl having from 1 to 20 carbon atoms.

2. The fluorinated dialcohol-diimine pro-ligand of claim 1 wherein both R² are the same and are phenyl groups unsubstituted or substituted or alkyl group, preferably methyl.

3. The fluorinated dialcohol-diimine pro-ligand of claim 2 wherein the phenyl groups are substituted with H, F or CF₃ at positions 2 and/or 3 and/or 4 and/or 5 and/or 6 or with isopropyl at positions 2 and 6.

4. The fluorinated dialcohol-diimine pro-ligand of any one of claims 1 to 3 wherein R³ and R⁴ are the same and are CF₃ or CₙF₂ₙ₊₁, preferably CF₃.

5. The fluorinated dialcohol-diimine pro-ligand of any one of the preceding claims wherein R⁵ and R⁶ are the same and are hydrogen.

6. A metallic complex resulting from the metallation reaction of the fluorinated dialcohol-diimine pro-ligand of any one of claims 1 to 5 with a metal salt of formula MX₄ wherein M is a metal group 4 of the Periodic Table, and wherein each X is the same or different and is halogen, alkyl or benzyl

7. The metallic complex of claim 6 wherein X is chlorine or methyl or benzyl

8. A method for preparing the metallic complex of claim 6 or claim 7 by the steps of:
a) providing the pro-ligand of any one of claims 1 to 5;
b) complexing it with metal salt or precursor of formula MX₄ wherein M and X are as defined in any one of claims 6 or 7.

9. An active catalyst system comprising the metallic complex of claim 6 or claim 7, an activating agent having an ionising action and optionally a cocatalyst.

10. The active catalyst of claim 9 wherein the activating agent is an aluminoxane or an activating support.

11. A method for oligomerising or homo- or co-polymerising ethylene or alpha-olefins that comprises the steps of:
a) injecting the active catalyst system of claim 9 or claim 10 into the reactor;
b) injecting the monomer and optional comonomer(s) simultaneously with or after step a);
c) maintaining under polymerisation conditions;
d) retrieving an oligomer or a polymer.
